# EUROPEAN PATENT SPECIFICATION

(11) **EP 1 388 331 B1**
(45) Date of publication and mention of the grant of the patent: **22.12.2004**
(21) Application number: 03077109.1
(22) Date of filing: 04.07.2003
(51) Int. Cl.: A61F 9/007

(54) **Liquefaction handpiece tip**
Spitze für ein Verflüssigungshandstück
Embout de pièce à main de liquéfaction

(30) Priority: 08.08.2002 US 214623
(43) Date of publication of application: 11.02.2004
(73) Proprietor: Alcon Inc., 6331 Hünenberg (CH)
(72) Inventor: Boukhny, Mikhail, Laguna Niguel, California 92677 (US)
(74) Representative: Hanna, Peter William Derek

(56) References cited:
- WO-A-01/30284
- US-A- 4 689 040
- US-A1- 2002 077 585

## Description

### Background of the Invention

This invention relates generally to the field of cataract surgery and more particularly to a handpiece tip for practicing the liquefaction technique of cataract removal.

The human eye in its simplest terms functions to provide vision by transmitting light through a clear outer portion called the cornea, and focusing the image by way of the lens onto the retina. The quality of the focused image depends on many factors including the size and shape of the eye, and the transparency of the cornea and lens.

When age or disease causes the lens to become less transparent, vision deteriorates because of the diminished light which can be transmitted to the retina. This deficiency in the lens of the eye is medically known as a cataract. An accepted treatment for this condition is surgical removal of the lens and replacement of the lens function by an artificial intraocular lens (IOL).

In the United States, the majority of cataractous lenses are removed by a surgical technique called phacoemulsification. During this procedure, a thin phacoemulsification cutting tip is inserted into the diseased lens and vibrated ultrasonically. The vibrating cutting tip liquifies or emulsifies the lens so that the lens may be aspirated out of the eye. The diseased lens, once removed, is replaced by an artificial lens.

A typical ultrasonic surgical device suitable for ophthalmic procedures consists of an ultrasonically driven handpiece, an attached cutting tip, and irrigating sleeve and an electronic control console. The handpiece assembly is attached to the control console by an electric cable and flexible tubes. Through the electric cable, the console varies the power level transmitted by the handpiece to the attached cutting tip and the flexible tubes supply irrigation fluid to and draw aspiration fluid from the eye through the handpiece assembly.

The operative part of the handpiece is a centrally located, hollow resonating bar or horn directly attached to a set of piezoelectric crystals. The crystals supply the required ultrasonic vibration needed to drive both the horn and the attached cutting tip during phacoemulsification and are controlled by the console. The crystal/horn assembly is suspended within the hollow body or shell of the handpiece by flexible mountings. The handpiece body terminates in a reduced diameter portion or nosecone at the body's distal end. The nosecone is externally threaded to accept the irrigation sleeve. Likewise, the horn bore is internally threaded at its distal end to receive the external threads of the cutting tip. The irrigation sleeve also has an internally threaded bore that is screwed onto the external threads of the nosecone. The cutting tip is adjusted so that the tip projects only a predetermined amount past the open end of the irrigating sleeve. Ultrasonic handpieces and cutting tips are more fully described in U.S. Pat. Nos. 3,589,363; 4,223,676; 4,246,902; 4,493,694; 4,515,583; 4,589,415; 4,609,368; 4,869,715; 4,922,902; 4,989,583; 5,154,694 and 5,359,996.

In use, the ends of the cutting tip and irrigating sleeve are inserted into a small incision of predetermined width in the cornea, sclera, or other location. The cutting tip is ultrasonically vibrated along its longitudinal axis within the irrigating sleeve by the crystal-driven ultrasonic horn, thereby emulsifying the selected tissue in situ. The hollow bore of the cutting tip communicates with the bore in the horn that in turn communicates with the aspiration line from the handpiece to the console. A reduced pressure or vacuum source in the console draws or aspirates the emulsified tissue from the eye through the open end of the cutting tip, the cutting tip and horn bores and the aspiration line and into a collection device. The aspiration of emulsified tissue is aided by a saline flushing solution or irrigant that is injected into the surgical site through the small annular gap between the inside surface of the irrigating sleeve and the cutting tip.

Recently, a new cataract removal technique has been developed that involves the injection of hot (approximately 45°C to 105°C) water or saline to liquefy or gellate the hard lens nucleus, thereby making it possible to aspirate the liquefied lens from the eye. Aspiration is conducted concurrently with the injection of the heated solution and the injection of a relatively cool solution, thereby quickly cooling and removing the heated solution. This technique is more fully described in U.S. Patent No. 5,616,120 (Andrew, et al.). The apparatus disclosed in the publication, however, heats the solution separately from the surgical handpiece. Temperature control of the heated solution can be difficult because the fluid tubes feeding the handpiece typically are up to two meters long, and the heated solution can cool considerably as it travels down the length of the tube.

U.S. Patent No. 5,885,243 (Capetan, et al.) discloses a handpiece having a separate pumping mechanism and resistive heating element. Such a structure adds unnecessary complexity to the handpiece.

U.S. Patent Nos. 5,989,212, 5,997,499, 6,110,162, 6,179,805, 6,196,989, 6,206,848, 6,287,274, 6,315,755, 6,331,171 and 6,398,759, all disclose various types of liquefaction handpieces and tips. These prior art tips do not allow the high pressure fluid stream to contact the cataractous material directly without leaving the tip and exposing the internal structures of the eye to the high pressure fluid stream. Document US-A-2002/0077585 discloses a liquefaction tip comprising the features recited in the preamble of claim 1.

A need continues to exist for a liquefaction tip that allows the material being removed to be directly exposed to the high pressure fluid while preventing the remaining eye structures from being exposed to the high pressure fluid stream.

### Brief Summary of the Invention

The present invention provides a liquefaction tip in accordance with the claim which follows. The invention improves on the prior art by providing a surgical liquefaction tip having an angled concave rim defining an arcuate or scalloped distal end. Such a construction allows the heated pulses of fluid produced by the liquefaction handpiece to strike the lens without being allowed to directly exit the tip.

Accordingly, one objective of the present invention is to provide a liquefaction tip for delivering pulses of fluid directly against the lens.

Another objective of the present invention is to provide a liquefaction tip for delivering pulses of fluid that do not directly enter the eye.

These and other advantages and objectives of the present invention will become apparent from the detailed description and claims that follow.

### Brief Description of the Drawings

FIG. 1 is a partial cross-sectional view of a prior art liquefaction handpiece tip.
FIG. 2 is a partial cross-sectional view of the liquefaction handpiece tip of the present invention.
FIG. 3 is a partial cross-sectional view of the liquefaction handpiece tip of the present invention similar to FIG. 2, but illustrating the location of the lens during use.

### Detailed Description of the Invention

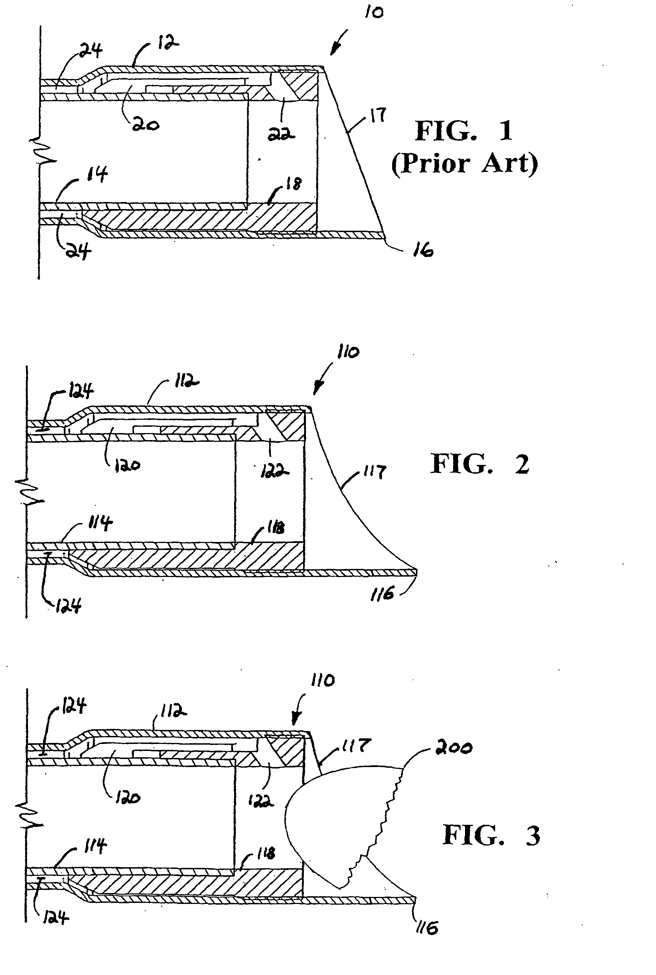

As best seen in FIG. 1, prior art liquefaction tip 10 generally consists of outer tube 12 surrounding and coaxial with inner tube 14A distal portionof outer tube 12 is flared or belled so as to allow nozzle 18 to be inserted between outer tube 12 and inner tube 14. Nozzle 18 contains fluid channel 20 that communicates with orifice 22. Nozzle 18 seals annular gap 24 between outer tube 12 and inner tube 14. Pressurized fluid flowing down annular gap 24 is forced into fluid channel 20, out orifice 22 and against inner tube 14 near distal tip 16. Such a construction prevents pressurized fluid from directly entering the eye, however, due to the straight configuration and angle of rim 17, lens material is restricted from entering outer tube 12 and being directly exposed to the fluid jet exiting orifice 22.

As best seen in FIG. 2, tip 110 of the present invention generally includes outer tube 112 surrounding and coaxial with inner tube 114. A distal portion of outer tube 112 is flared or belled so as to allow nozzle 118 to be inserted between outer tube 112 and inner tube 114. Nozzle 118 contains fluid channel 120 that communicates with orifice 122. Nozzle 118 seals annular gap 124 between outer tube 112 and inner tube 114. Pressurized fluid flowing down annular gap 124 is forced into fluid channel 120, out orifice 122 and against inner tube 114 near distal tip 116. Such a construction prevents pressurized fluid from directly entering the eye. Rim 117 is cut in a concave or inwardly arcuate shape. Such a construction allows pieces of lens material 200 to enter distal tip 116 and be exposed directly to the fluid jet exiting orifice 122, as best seen in FIG. 3.

This description is given for purposes of illustration and explanation. It will be apparent to those skilled in the relevant art that changes and modifications may be made to the invention described above without departing from its scope For example, it will be recognized by those skilled in the art that the present invention may be combined with ultrasonic and/or rotating cutting tips to enhance performance.

## Claims

1. A tip (110) for a liquefaction handpiece, comprising;
a) an inner tube (114) coaxially mounted within an outer tube (112) so as to form an annular gap (124) between the inner tube and the outer tube, the outer tube defining an angled rim having a distal tip (116);
b) a nozzle (118) at a distal end of the annular gap, inserted between the outer tube and the inner tube;
c) at least one discharge orifice (122) in the nozzle, the orifice in fluid communication with the annular gap, such that a fluid jet exiting the orifice is directed against the inner tube near said distal tip,
**characterized in that** said rim (117) is formed with a concave configuration.

## Patentansprüche

1. Spitze (110) für ein Verflüssigungshandstück, das folgendes umfaßt:
a) eine innere Röhre (114), die koaxial in einer äußeren Röhre (112) montiert ist, so daß ein ringförmiger Spalt (124) zwischen der inneren Röhre und der äußeren Röhre gebildet wird, wobei die äußerer Röhre einen abgewinkelten Rand mit einer distalen Spitze (116) festlegt;
b) eine Düse (118) an einem distalen Ende des ringförmigen Spalts, die zwischen der äußeren Röhre und der inneren Röhre eingesetzt ist;
c) wenigstens eine Austrittsmündung (122) in der Düse, wobei die Mündung in Fluidkommunikation mit dem ringförmigen Spalt steht, so daß ein Fluidstrahl, der die Mündung verläßt, entgegen der inneren Röhre bei dem distalen Ende gerichtet ist,
**dadurch gekennzeichnet, daß** der Rand (117) in einer konkaven Konfiguration ausgebildet ist.

## Revendications

1. Embout (110) pour une pièce à main de liquéfaction, comprenant :
a) un tube intérieur (114) monté coaxialement à l'intérieur d'un tube extérieur (112) de façon à former un espace annulaire (124) entre le tube intérieur et le tube extérieur, le tube extérieur définissant une collerette oblique présentant une pointe distale (116) ;
b) une buse (118) au niveau d'une extrémité distale de l'espace annulaire, insérée entre le tube extérieur et le tube intérieur ;
c) au moins un orifice de vidage (122) dans la buse, l'orifice étant en communication pour fluide avec l'espace annulaire, de sorte qu'un jet de fluide sortant par l'orifice est orienté contre le tube intérieur à proximité de ladite pointe distale,
**caractérisé en ce que** ladite collerette (117) est formée avec une configuration concave.
